# EUROPEAN PATENT APPLICATION

(11) **EP 0 967 213 A1**
(43) Date of publication of application: **29.12.1999**
(21) Application number: 99304893.3
(22) Date of filing: 22.06.1999
(51) Int. Cl.: C07D 473/40

(54) **Process for preparing tetraalkylammonium salt of 6-halogenopurine derivative**

(30) Priority: 23.06.1998 JP 17600498
(71) Applicant: SUMIKA FINE CHEMICALS Co., Ltd., Nishi Yodogawa-ku, Osaka-shi, Osaka (JP)
(72) Inventor: Hayashi, Taketo, c/o Sumika Fine Chemicals Co. Ltd, Osaka-shi, Osaka (JP); Nishiwaki, Kenji, Sumika Fine Chemicals Co. Ltd., Osaka-shi, Osaka (JP)
(74) Representative: Marchant, James Ian

(57) **Abstract**

A process for preparing a tetraalkylammonium salt of a 6-halogenopurine derivative, represented by the formula: 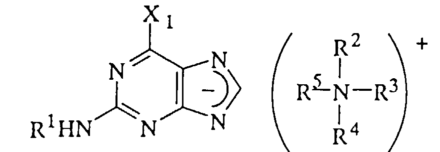 wherein R¹ is hydrogen, formyl group or acetyl group; each of R², R³, R⁴ and R⁵ is independently a C₁₋₁₀ alkyl group; X₁ is chlorine, bromine or iodine, comprising reacting a 6-halogenopurine derivative represented by the formula: 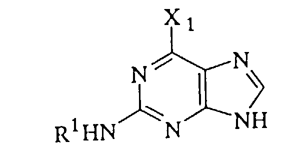 wherein R¹ and X₁ are the same as defined above, or a salt thereof with a tetraalkylammonium salt represented by the formula: 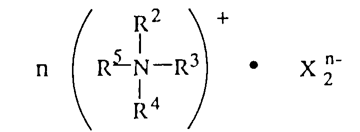 wherein R², R³, R⁴ and R⁵ are the same as defined above; X₂ is chlorine, bromine, iodine, an inorganic acid residue or an organic acid residue; n is an integer of 1 to 3. The tetraalkylammonium salt of a 6-halogenopurine derivative is useful as intermediates for preparation of antiviral agents.

## Description

### BACKGROUND OF THE INVENTION

### Field of the Invention

The present invention relates to a process for preparing a tetraalkylammonium salt of a 6-halogenopurine derivative. More specifically, the present invention relates to a process for preparing a tetraalkylammonium salt of a 6-halogenopurine derivative, which is useful as intermediates for preparation of antiviral agents.

### Discussion of the Related Art

Conventionally, as a process for preparing a tetraalkylammonium salt of a 6-halogenopurine derivative, there has been proposed a process for reacting a 6-halogenopurine derivative with an alkylammonium hydroxide in a solvent such as water, ethanol or methylene chloride [Japanese Patent Laid-Open No. Hei 6-206879 and *J. Org. Chem.,* **60**, 2902-2905 (1995)].

However, there are some defects in these processes such that these processes necessitate an expensive alkylammonium hydroxide.

Accordingly, it has been expected to develop a process for preparing a tetraalkylammonium salt of a 6-halogenopurine derivative on an industrial scale without using an alkylammonium hydroxide.

In view of the above problems, an object of the present invention is to provide a process for industrially and advantageously preparing a tetraalkylammonium salt of a 6-halogenopurine derivative without using an alkylammonium hydroxide.

This and other objects of the present invention will be apparent from the following description.

### SUMMARY OF THE INVENTION

In accordance with the present invention, there is provided a process for preparing a tetraalkylammonium salt of a 6-halogenopurine derivative, represented by the formula (III): 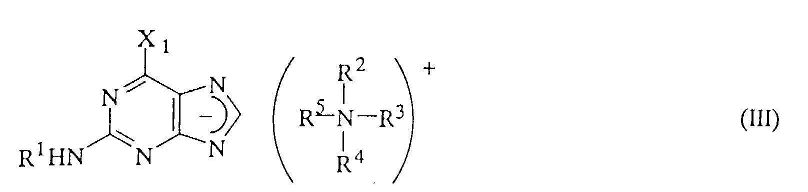 wherein R¹ is hydrogen atom, formyl group or acetyl group; each of R², R³, R⁴ and R⁵ is independently a substituted or unsubstituted, linear or branched alkyl group having 1 to 10 carbon atoms; X₁ is chlorine atom, bromine atom or iodine atom,
comprising reacting a 6-halogenopurine derivative represented by the formula (I): 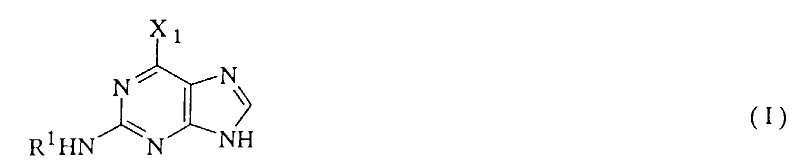 wherein R¹ and X₁ are the same as defined above, or a salt thereof with a tetraalkylammonium salt represented by the formula (II): 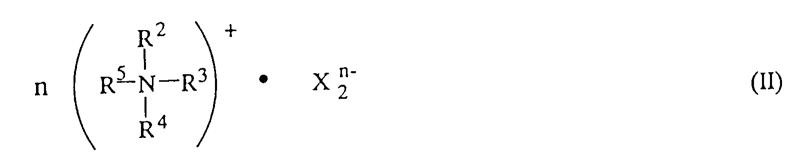 wherein R², R³, R⁴ and R⁵ are the same as defined above; X₂ is chlorine, bromine, iodine, an inorganic acid residue or an organic acid residue; n is an integer of 1 to 3.

### DETAILED DESCRIPTION OF THE INVENTION

According to the present invention, as described above, the tetraalkylammonium salt of a 6-halogenopurine derivative, represented by the formula (III): 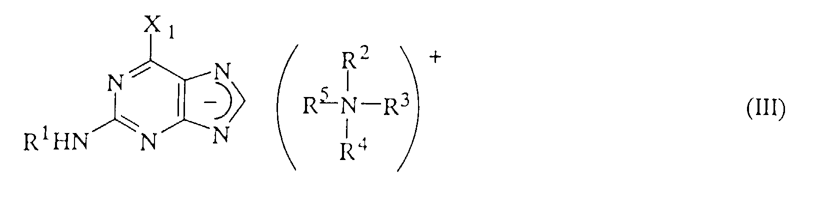 wherein R¹ is hydrogen atom, formyl group or acetyl group; each of R², R³, R⁴ and R⁵ is independently a substituted or unsubstituted, linear or branched alkyl group having 1 to 10 carbon atoms; X₁ is chlorine atom, bromine atom or iodine atom,
can be prepared by reacting a 6-halogenopurine derivative represented by the formula (I): 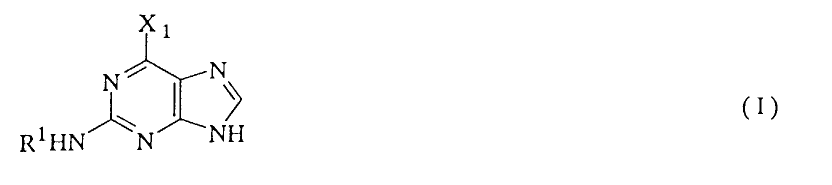 wherein R¹ and X₁ are the same as defined above, or a salt thereof with a tetraalkylammonium salt represented by the formula (II): 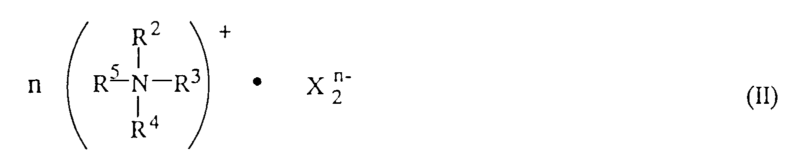 wherein R², R³, R⁴ and R⁵ are the same as defined above; X₂ is chlorine, bromine, iodine, an inorganic acid residue or an organic acid residue; n is an integer of 1 to 3.

In the 6-halogenopurine derivative represented by the formula (I), R¹ is hydrogen atom, formyl group or acetyl group.

X₁ is chlorine atom, bromine atom or iodine atom.

The salt of the 6-halogenopurine derivative represented by the formula (I) includes, for instance, acetates, hydrochlorides, hydroiodates, sodium salts, potassium salts, and the like.

The tetraalkylammonium salt represented by the formula (II) is an inexpensive and readily commercially available compound.

In the formula (II), each of R², R³, R⁴ and R⁵ is independently a substituted or unsubstituted, linear or branched alkyl group having 1 to 10 carbon atoms.

The linear or branched alkyl group having 1 to 10 carbon atoms includes, for instance, methyl group, ethyl group, n-propyl group, isopropyl group, n-butyl group, isobutyl group, sec-butyl group, tert-butyl group, n-pentyl group, n-hexyl group, n-heptyl group, n-octyl group, n-nonyl group, n-decyl group, and the like.

The alkyl group may have one to three substituents selected from the group consisting of an alkoxy group having 1 to 6 carbon atoms or an aryl group having 6 to 12 carbon atoms.

The alkoxy group having 1 to 6 carbon atoms includes, for instance, methoxy group, ethoxy group, n-propoxy group, isopropoxy group, n-butoxy group, isobutoxy group, sec-propoxy group, tert-propoxy group, n-pentyloxy group, n-hexyloxy group, and the like.

The aryl group having 6 to 12 carbon atoms includes phenyl group which may have one to three substituents selected from the group consisting of an alkyl group having 1 to 6 carbon atoms, an alkoxy group having 1 to 6 carbon atoms, and a halogen atom. The alkyl group having 1 to 6 carbon atoms includes, for instance, methyl group, ethyl group, n-propyl group, isopropyl group, n-butyl group, isobutyl group, sec-butyl group, tert-butyl group, n-pentyl group, n-hexyl group, and the like. The alkoxy group having 1 to 6 carbon atoms includes, for instance, methoxy group, ethoxy group, n-propoxy group, isopropoxy group, n-butoxy group, isobutoxy group, sec-butoxy group, tert-butoxy group, n-pentyloxy group, n-hexyloxy group, and the like.

The halogen atom includes fluorine atom, chlorine atom, bromine atom and iodine atom.

X₂ is chlorine, bromine, iodine, an inorganic acid residue or an organic acid residue, and n is an integer of 1 to 3.

The inorganic acid residue includes, for instance, SO₄, HSO₄, H₂PO₄, HPO₄, PO₄, NO₃, and the like. Incidentally, when the inorganic acid residue is HSO₄, H₂PO₄ or NO₃, n is 1; when the inorganic acid residue is SO₄ or HPO₄, n is 2; and when the inorganic acid residue is PO₄, n is 3.

The organic acid residue includes, for instance, HCOO, CH₃COO, CH₃SO₃, p-CH₃C₆H₄SO₃, and the like. In all these exemplified above, n is 1.

It is desired that the amount of the tetraalkylammonium salt represented by the formula (II) is at least one mol per one mol of the 6-halogenopurine derivative represented by the formula (I), from the viewpoint of giving a 1:1 molar ratio of the ions of the 6-halogenopurine derivative to the tetraalkylammonium ions, and that the amount is at most 5 mol, preferably at most 2 mol per one mol of the 6-halogenopurine derivative represented by the formula (I), from the viewpoint of economic advantages.

The reaction of the 6-halogenopurine derivative represented by the formula (I) or a salt thereof with the tetraalkylammonium salt represented by the formula (II) can be carried out by, for instance, adding the 6-halogenopurine derivative represented by the formula (I) or a salt thereof, and the tetraalkylammonium salt represented by the formula (II) to an alcohol solution of an alkali metal hydroxide.

The alkali metal hydroxide includes, for instance, sodium hydroxide, potassium hydroxide, and the like.

It is desired that the amount of the alkali metal hydroxide is at least one mol per one mol of the 6-halogenopurine derivative represented by the formula (I), from the viewpoint of giving a 1:1 molar ratio of the ions of the 6-halogenopurine derivative to the tetraalkylammonium ions, and that the amount is at most 5 mol, preferably at most 2 mol per one mol of the 6-halogenopurine derivative represented by the formula (I), from the viewpoint of economic advantages. However, it is desired that the amount of the alkali metal hydroxide is at most equimolar to the amount of the tetraalkylammonium salt, from the viewpoint of quality of the resulting tetraalkylammonium salt of a 6-halogenopurine derivative.

The alcohol includes, for instance, monohydric alcohols having 1 to 10 carbon atoms, such as methanol, ethanol, 2-propanol, n-butanol, n-pentanol, n-hexanol, n-heptanol, n-octanol, and the like.

It is desired that the amount of the alcohol is adjusted so that the amount of the alkali metal hydroxide is usually 1 to 30 parts by weight, based on 100 parts by weight of the alcohol.

When the 6-halogenopurine derivative represented by the formula (I) or a salt thereof is reacted with the tetraalkylammonium salt represented by the formula (II), the reaction temperature and atmosphere are not limited to specified ones. For instance, it is desired that the reaction temperature is usually 5° to 60°C or so, and that the atmosphere may be air, or an inert gas, such as nitrogen gas.

The reaction time cannot be absolutely determined because the reaction time can differ depending upon the reaction temperature, and the like. The reaction time is usually from 30 minutes to 4 hours or so. Since the metal hydroxides disappear with the progress of the reaction, and the pH of the reaction mixture is lowered, the termination of reduction can be confirmed, for instance, by the determination of a pH of the reaction mixture.

After the termination of the reaction, the resulting tetraalkylammonium salt of a 6-halogenopurine derivative can be isolated by usual procedures such as concentration, precipitation, filtration, drying, washing, and the like.

The resulting tetraalkylammonium salt of a 6-halogenopurine derivative, represented by the formula (III) is a compound which can be suitably used as an intermediate for an antiviral agent disclosed in, for instance, *J. Org. Chem.*, **60**, 2902-2905 (1995), *J. Org. Chem.*, **62**, 1580-1581 (1997), and the like.

### EXAMPLES

The present invention will be more specifically described by the following examples, without intending to restrict the scope or spirit of the present invention thereto.

### Example 1

To 129 ml of methanol was added 15.8 g (0.270 mol) of 96% potassium hydroxide at room temperature. Thereafter, 88.9 g (0.276 mol) of tetrabutylammonium bromide was added thereto, and 70.6 g (0.270 mol) of 2-amino-6-iodopurine was added thereto at room temperature. The resulting mixture was stirred for 30 minutes, and 486 ml of 4-methyl-2-pentanone was then added thereto. Thereafter, the solvent was distilled off under reduced pressure, and 510 ml of 4-methyl-2-pentanone and 3.7 g of activated charcoal were then added thereto. After stirring the mixture for 30 minutes, the activated charcoal was removed therefrom, to give a 4-methyl-2-pentanone solution of tetrabutylammonium salt of 2-amino-6-iodopurine. The solvent was distilled off again under reduced pressure from the resulting 4-methyl-2-pentanone solution, and the residue was cooled to room temperature. The precipitated crystals were collected by filtration at room temperature, and washed with 383 ml of ethyl acetate. The resulting wet crystals were dried under reduced pressure, to give 115.3 g (0.229 mol) of white crystals of a tetrabutylammonium salt of 2-amino-6-iodopurine (yield: 85.0%).

The resulting white crystals had the following physical properties.
(1) Melting point: 114°C
(2) Elemental Analysis (C₂₁H₃₉IN₆)

| | | | | |
|---|---|---|---|---|
| Calculated Value | C 50.20%; | H 7.82%; | I 25.26%; | N 16.72% |
| Found Value | C 50.32%; | H 7.96%; | I 25.33%; | N 16.87% |

### Example 2

To 48 ml of ethanol was added 5.8 g (0.100 mol) of 96% potassium hydroxide at room temperature. Thereafter, 17.4 g (0.105 mol) of tetraethylammonium chloride was added thereto, and 26.1 g (0.100 mol) of 2-amino-6-iodopurine was added thereto at room temperature. The resulting mixture was stirred for 30 minutes, and 180 ml of 4-methyl-2-pentanone was then added thereto, and the precipitated crystals were immediately separated therefrom by filtration, to give a 4-methyl-2-pentanone solution of a tetraethylammonium salt of 2-amino-6-iodopurine. The resulting 4-methyl-2-pentanone solution was concentrated under reduced pressure until no solvent was distilled off. To the residue was added 150 ml of ethyl acetate with stirring the residue at high speed. The precipitated crystals were collected by filtration at room temperature, and washed with 70 ml of ethyl acetate. The resulting wet crystals were dried under reduced pressure, to give 27.5 g (0.070 mol) of white crystals of a tetraethylammonium salt of 2-amino-6-iodopurine (yield: 70.5%).

The resulting white crystals had the following physical properties.
(1) Melting point: 177°C (decomposed)
(2) Elemental Analysis (C₁₃H₂₃IN₆)

| | | | | |
|---|---|---|---|---|
| Calculated Value | C 40.01%; | H 5.94%; | I 32.52%; | N 21.53% |
| Found Value | C 39.98%; | H 5.91%; | I 32.47%; | N 21.52% |

### Example 3

To 48 ml of methanol was added 5.8 g (0.100 mol) of 96% potassium hydroxide at room temperature. Thereafter, 23.9 g (0.105 mol) of benzyltriethylammonium chloride was added thereto, and 26.1 g (0.100 mol) of 2-amino-6-iodopurine was added thereto at room temperature. The resulting mixture was stirred for 30 minutes, and 180 ml of 4-methyl-2-pentanone was then added thereto, and the precipitated crystals were collected by filtration, to give a 4-methyl-2-pentanone solution of a benzyltriethylammonium salt of 2-amino-6-iodopurine. The resulting 4-methyl-2-pentanone solution was concentrated under reduced pressure until no solvent was distilled off. To the residue was added 150 ml of ethyl acetate with stirring the residue at high speed. The precipitated crystals were collected by filtration at room temperature, and washed with 70 ml of ethyl acetate. The resulting wet crystals were dried under reduced pressure, to give 37.2 g (0.082 mol) of white crystals of a benzyltriethylammonium salt of 2-amino-6-iodopurine (yield: 82.2%).

The resulting white crystals had the following physical properties.
(1) Melting point: 146°C
(2) Elemental Analysis (C₁₈H₂₅IN₆)

| | | | | |
|---|---|---|---|---|
| Calculated Value | C 47.80%; | H 5.57%; | I 27.88%; | N 18.58% |
| Found Value | C 47.83%; | H 5.69%; | I 28.00%; | N 18.66% |

### Example 4

To 48 ml of methanol was added 4.0 g (0.100 mol) of 99% sodium hydroxide at room temperature. Thereafter, 33.8 g (0.105 mol) of tetrabutylammonium bromide was added thereto, and 16.7 g (0.100 mol) of 2-amino-6-chloropurine was added thereto at room temperature. The resulting mixture was stirred for 30 minutes, and 180 ml of 4-methyl-2-pentanone was then added thereto, and the precipitated crystals were removed therefrom, to give a 4-methyl-2-pentanone solution of a tetrabutylammonium salt of 2-amino-6-chloropurine. The resulting 4-methyl-2-pentanone solution was concentrated under reduced pressure until no solvent was distilled off. To the residue was added 150 ml of ethyl acetate with stirring the residue at high speed. The precipitated crystals were collected by filtration at room temperature, and washed with 70 ml of ethyl acetate. The resulting wet crystals were dried under reduced pressure, to give 35.1 g (0.085 mol) of white crystals of a tetrabutylammonium salt of 2-amino-6-chloropurine (yield: 85.4%).

The resulting white crystals had the following physical properties.
(1) Melting point: 85°C
(2) Elemental Analysis (C₂₁H₃₉ClN₆)

| | | | | |
|---|---|---|---|---|
| Calculated Value | C 61.37%; | H 9.56%; | Cl 18.63%; | N 20.45% |
| Found Value | C 61.41%; | H 9.57%; | Cl 18.69%; | N 20.52% |

### Example 5

To 149 ml of 2-propanol was added 5.8 g (0.100 mol) of 96% potassium hydroxide at room temperature. Thereafter, 17.4 g (0.105 mol) of tetraethylammonium chloride was added thereto, and the precipitated crystals were removed therefrom. To the resulting 2-propanol solution was added 17.0 g (0.100 mol) of 2-amino-6-chloropurine at room temperature. The resulting mixture was stirred for 30 minutes, and 180 ml of 4-methyl-2-pentanone was then added thereto. The precipitated crystals were collected by filtration at room temperature, and washed with 70 ml of ethyl acetate. The resulting wet crystals were dried under reduced pressure, to give 24.7 g (0.083 mol) of white crystals of a tetraethylammonium salt of 2-amino-6-chloropurine (yield: 82.7%).

The resulting white crystals had the following physical properties.
(1) Melting point: 160°C (decomposed)
(2) Elemental Analysis (C₁₃H₂₃ClN₆)

| | | | | |
|---|---|---|---|---|
| Calculated Value | C 52.25%; | H 7.76%; | Cl 11.86%; | N 28.12% |
| Found Value | C 52.28%; | H 7.90%; | Cl 11.83%; | N 28.11% |

### Example 6

To 48 ml of methanol was added 4.4 g (0.075 mol) of 96% potassium hydroxide at room temperature. Thereafter, 24.2 g (0.075 mol) of tetrabutylammonium bromide was added thereto, and 12.9 g (0.050 mol) of 2-formylamino-6-chloropurine acetate was added thereto. The resulting mixture was stirred for 30 minutes, and 180 ml of 4-methyl-2-pentanone was then added thereto. Thereafter, the solvent was distilled off under reduced pressure, and 180 ml of 4-methyl-2-pentanone and 3.7 g of activated charcoal were then added thereto. After stirring the mixture for 30 minutes, the activated charcoal was removed therefrom, to give a 4-methyl-2-pentanone solution of a tetrabutylammonium salt of 2-formylamino-6-chloropurine. The 4-methyl-2-pentanone solution was concentrated until no solvent was distilled off. To the residue was added 150 ml of ethyl acetate with stirring the residue at high speed. The precipitated crystals were collected by filtration at room temperature, and washed with 70 ml of ethyl acetate. The resulting wet crystals were dried under reduced pressure, to give 11.9 g (0.027 mol) of white crystals of a tetrabutylammonium salt of 2-formylamino-6-chloropurine (yield: 54.0%).

The resulting white crystals had the following physical properties.
(1) Melting point: 104.3°C
(2) Elemental Analysis (C₂₂H₃₉ClN₆O)

| | | | | |
|---|---|---|---|---|
| Calculated Value | C 60.18; | H 8.95%; | Cl 8.08%; | N 19.14% |
| Found Value | C 60.12%; | H 8.99%; | Cl 8.12%; | N 19.17% |

It is clear from the above results that according to the processes of Examples 1 to 6, the tetraalkylammonium salt of a 6-halogenopurine derivative can be easily prepared by using an inexpensive and readily available tetraalkylammonium salt.

According to the present invention, there can be exhibited such remarkable effects that the tetraalkylammonium salt of a 6-halogenopurine derivative can be industrially and advantageously prepared without using the conventionally used expensive alkylammonium hydroxide.

The present invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

## Claims

1. A process for preparing a tetraalkylammonium salt of a 6-halogenopurine derivative, represented by the formula (III): 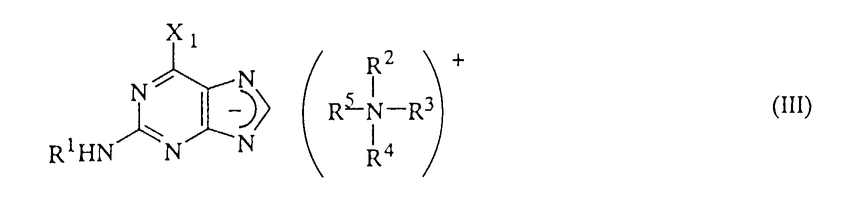 wherein R¹ is hydrogen atom, formyl group or acetyl group; each of R², R³, R⁴ and R⁵ is independently a substituted or unsubstituted, linear or branched alkyl group having 1 to 10 carbon atoms; X₁ is chlorine atom, bromine atom or iodine atom,
comprising reacting a 6-halogenopurine derivative represented by the formula (I): 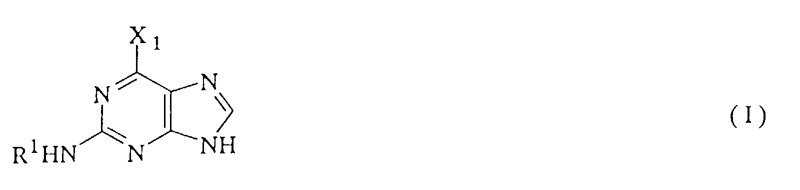 wherein R¹ and X₁ are the same as defined above,
or a salt thereof with a tetraalkylammonium salt represented by the formula (II): 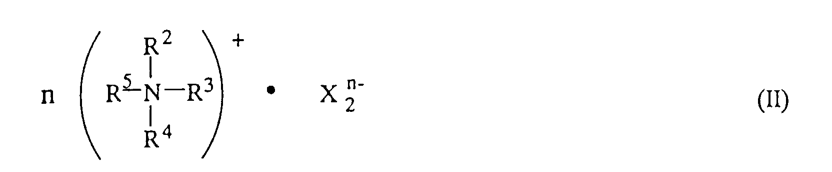 wherein R², R³, R⁴ and R⁵ are the same as defined above; X₂ is chlorine, bromine, iodine, an inorganic acid residue or an organic acid residue; n is an integer of 1 to 3.

2. The process according to claim 1, wherein the amount of the tetraalkylammonium salt is 1 to 5 mol per one mol of the 6-halogenopurine derivative or a salt thereof.

3. The process according to claim 1, wherein the 6-halogenopurine derivative or a salt thereof is reacted with the tetraalkylammonium salt in an alcohol solution of an alkali metal hydroxide.

4. The process according to claim 3, wherein the alkali metal hydroxide is sodium hydroxide or potassium hydroxide.

5. The process according to claim 3, wherein the alcohol is a monohydric alcohol having 1 to 10 carbon atoms.

6. The process according to claim 5, wherein the monohydric alcohol is methanol, ethanol, 2-propanol, n-butanol, n-pentanol, n-hexanol, n-heptanol or n-octanol.
